(19) Europäisches Patentamt / European Patent Office / Office européen des brevets

(11) **EP 4 302 792 A1**

(12) **EUROPEAN PATENT APPLICATION**

(43) Date of publication:
**10.01.2024 Bulletin 2024/02**

(21) Application number: **22183025.0**

(22) Date of filing: **05.07.2022**

(51) International Patent Classification (IPC):
**A61M 1/06** (2006.01)

(52) Cooperative Patent Classification (CPC):
**A61M 1/06; A61M 1/067; A61M 1/0693;**
A61M 2205/332; A61M 2205/3365; A61M 2209/088

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **DE VREEDE, Jasper**
  **Eindhoven (NL)**
- **VAN BENTUM, Jesper William**
  **Eindhoven (NL)**
- **VAN DER KOOI, Johannes Tseard**
  **Eindhoven (NL)**

(74) Representative: **Philips Intellectual Property & Standards**
**High Tech Campus 52**
**5656 AG Eindhoven (NL)**

(54) **MOVEMENT BASED ADJUSTMENTS TO BREAST PUMP**

(57)     A system for controlling a holding force of a breast pump system exerted on a user during operation. A controller is used to receive and analyze motion data coming from sensors measuring the motion intensity and/or tilt of the user when wearing the breast pump system. If the motion data indicates a change in the motion intensity and/or the tilt of the user, one or more parameters of the breast pump system are adapted, during operation, to adapt the holding force exerted on the user.

FIG. 2

## Description

FIELD OF THE INVENTION

The invention relates to the field of breast pumps.

BACKGROUND OF THE INVENTION

The best source of nutrition for a baby is human milk given by a breastfeeding mother. The world health organization recommends to breast feed for at least one year, preferably longer. However, mothers often go back to work after only several weeks or months. To provide the best nutrition for their babies, mothers usually express milk using a breast pump. The expressed milk can be stored and given to the baby at a later time and/or by somebody else.

Recently, there has been a trend towards wearable breast pumps. These devices have a smaller form factor and can therefore fit in the bra of the user. With a wearable breast pump, women can ideally pump anytime and anywhere, regardless of the activity they are doing. In order to pump correctly during such activities, it is important that the wearable breast pump stays in place without leakage and/or user adjustments.

With the addition of a wearable breast pump, women have more freedom to move during a pump session. While freedom of movement brings many benefits to the user, it can also lead to malfunctioning and leakage of the breast pump due to sudden movements by the user. For example, when a woman goes on a walk, the breast pump may slowly shift due to the vibrations of the walk. This might lead to an incorrect nipple position, which makes pumping less effective, creates discomfort and, potentially, nipple damage, or increases the likelihood of leakage, which also makes pumping less effective.

Thus, there is a need to prevent the breast pump from shifting (slowly or abruptly) from the correct placement due to movements of the user and leakage of the pump due to movements of the user. In order to pump correctly during activities, it is important that the wearable breast pump stays in place without leakage and/or user adjustments.

SUMMARY OF THE INVENTION

The invention is defined by the claims.

According to examples in accordance with an aspect of the invention, there is provided a system for controlling a holding force of a breast pump system exerted on a user during operation, the system comprising a controller configured to:

> receive and analyze motion data of the user wearing the breast pump system, the motion data being indicative of a motion intensity and/or a tilt of the user;

and
adapt one or more parameters of the breast pump system during operation to adapt the holding force if the motion data indicates a change in motion intensity and/or tilt of the user.

The breast pump system may comprise a breast pump and means for holding the breast pump on the user (e.g. a smart bra). In an example, the straps of a smart bra are adaptable thereby to increase the holding force of the breast pump system exerted on the user during operation. The holding force is a force generated by, or induced by, one or more components of the breast pump system for holding the breast pump system securely against the user during operation of the breast pump in the breast pump system.

In general, the means for holding the breast pump on the user are used for ensuring the breast pump on the user whilst it is operational. It may be adaptable (i.e. via one or more of the parameters) such as a smart bra or it may not be (e.g. a conventional bra).

The holding force could be exerted on the user by the means for holding the breast pump (e.g. via a clamping-like force of a smart bra on the user) or by the breast pump itself.

In another embodiment, the breast pump system may not comprise means for holding the breast pump on the user. In these cases, the breast pump may be adapted to fit in a bra (or similar) of a user.

In an embodiment, the holding force comprises a suction force induced by the negative pressures (relative to ambient) of the breast pump used to extract milk.

In essence, this provides an adaptable breast pump which enables the breast pump to increase the suction force between the breast pump and the user when the user is moving. This reduces the likelihood of the breast pump shifting from its correct placement and prevents potential leakage of the pump due to the user's movement without the need to unduly increase the suction force at all times, thereby also improving the comfort of the breast pump as well as nipple and breast health.

The suction force is a force applied on the breast pump towards the user. The suction force is provided by a pressure difference between the volume formed between the breast pump and the user and atmospheric pressure. The suction force may refer to a minimum suction force provided by the breast pump over time. Alternatively, or additionally, the suction force could refer to a time-averaged force (e.g. averaged over more than two seconds)

The motion data may be received directly from a sensor unit or from a data storage system (e.g. computer memory).

The motion data may be analyzed by, for example, comparing it to pre-defined thresholds or inputting it into a pre-defined relationship between the motion data and the parameters. A table of corresponding motion data values and parameter values could also be used.

The change in motion intensity may refer to the acceleration (linear and/or rotational) of the user. The tilt of the user may refer to the orientation of the user with respect to gravity. Preferably, the tilt of the user refers to the orientation of the user's breasts and/or the breast pump (worn by the user) with respect to gravity.

The motion data is obtained whilst the breast pump is operating on the user.

The parameters are parameters which affect the operation of the breast pump. The parameter may be chosen such that a change in any one parameter affects the pressure cycle of the breast pump and/or the suction surface.

The adaptation in the parameters may be chosen such that the resulting suction force is positively correlated to (or in direct variation to) the change in motion intensity and/or tilt (i.e. when the motion intensity and/or the tilt go up, so does the suction force, and vice-versa).

The breast pump system may be configured to operate based on the one or more parameters. The breast pump system may generally be configured to increase the holding force (e.g. the suction force) of the breast pump when the measured motion increases (e.g. above a threshold).

The one or more parameters may affect the suction force of the breast pump. Thus, the suction force is dependent on one or more of the parameters of the breast pump (e.g. maximum negative pressure, baseline pressure, time spent on the maximum negative pressure, time spent on the negative pressure etc.).

The suction force is generally indicative of the magnitude of the forces generated by the pressure difference between the cavity between the breast pump and the user and the area outside the cavity. This pressure difference induces forces which, at least partially, keep the breast pump connected to the user. As such, an increase in the suction force is indicative of an increase in the forces keeping the breast pump connected to the user. In summary, the purpose of increasing the suction force (or even the holding force) is to increase the forces connecting the breast pump to the user thereby reducing the likelihood of the breast pump slipping or disconnecting during operation).

The logic used when adapting the parameters of the breast pump may be based on discrete steps where the parameters are adapted at particular measured motion values. For example, the relationship may comprise a table with different motion values and their corresponding parameters. Alternatively, the logic may be based on a continuous relationship (e.g. a continuous function) which adapts the parameters as a function of the motion data.

The motion data is representative of the motion of the user and may comprise one or more motion values (e.g., linear acceleration, angular acceleration, orientation etc.). The motion data may also comprise one or more motion values over time.

The controller may be configured to adapt one or more parameters of the breast pump system during operation to increase the holding force if the motion data indicates an increase in motion intensity of the user and/or an increase in the tilt of the user relative to gravity.

The controller may be configured to adapt one or more parameters of the breast pump system during operation to decrease the holding force if the motion data indicates a decrease in motion intensity of the user and/or a decrease in the tilt of the user relative to gravity.

The breast pump system may operate by cycling the breast pump between a minimum absolute pressure and a baseline pressure and wherein, in adapting one or more of the parameters of the breast pump system, the controller is configured to adapt the baseline pressure. In other words, the breast pump may operate by cycling between a maximum pressure difference and a baseline pressure difference (relative to ambient pressure).

The minimum absolute pressure is the pressure value generated by the breast pump which is further from ambient pressure. The minimum absolute pressure may be referred to as a maximum under-pressure (i.e. below ambient pressure).

Breast pumps typically cycle between a maximum pressure difference and a baseline pressure difference to mimic the sucking motion of an infant. The pressures providing these pressure differences may be adaptable parameters of the breast pump. At the baseline pressure, the breast pump is more likely to disconnect itself from the user as there is a smaller pressure difference between the baseline pressure and the ambient pressure.

The baseline pressure is the pressure value closest to atmospheric pressure in the pressure cycle (causing the lowest pressure difference), thereby providing the baseline pressure difference. The minimum absolute pressure is the lowest pressure in the pressure cycle (causing the highest pressure difference), thereby providing the maximum pressure difference.

Thus, adapting the baseline pressure can improve the stability of the connection between the breast pump and the user. The baseline pressure can be adapted to increase the suction force of the breast pump

In adapting the baseline pressure, the controller may be configured to adapt a magnitude of the baseline pressure to increase an absolute pressure difference between ambient pressure and the baseline pressure.

For example, decreasing the magnitude of the baseline pressure increases the pressure difference between the ambient pressure and the baseline pressure in the breast pump, thereby providing a higher baseline pressure difference. It is noted that the pressure within the breast pump is lower than ambient pressure as the pressure difference provides a force on the breast pump, thereby providing the suction force on the breast pump towards the user. Thus, decreasing the magnitude of the baseline pressure increases the baseline pressure difference and therefore increases the suction force on the breast pump, resulting in a stronger connection between the breast pump and the user.

In adapting the baseline pressure, the controller may be configured to decrease a time spent at the baseline pressure during a cycle.

Decreasing the time spent at the baseline pressure reduces the time period at which the breast pump is more likely to disconnect from the user. This reduction in time increases the (mean) suction force over time. The decrease in time spent at the baseline pressure also decreases the probability that a high acceleration force will occur when the pump is operating at the baseline pressure.

The controller may be configured to adapt one or more of the parameters of the breast pump system when the motion data indicates that the motion intensity of the user exceeds a motion intensity threshold and/or that the tilt of the user relative to gravity exceeds a tilt threshold.

The controller may be further configured to predict a change in motion intensity and/or a change in tilt based on the motion data and adapt one or more of the parameters of the breast pump system pre-emptively based on the predicted change in motion intensity and/or the predicted change in tilt.

Current measured motion, or the evolution of measured motion over time, can be used to predict what the future motion could be. For example, a gradual increase in speed may indicate the user is walking (or performing other activities involving motion). Thus, the parameters may be adapted to increase the suction force to anticipate more rigorous motion after a relatively small measured motion.

This may reduce the likelihood of the breast pump system not adapting quickly enough and therefore [partially] disconnecting from the user due to a sudden motion of the user.

The controller may be further configured to input the motion data into a pattern recognition algorithm and adapt one or more of the parameters of the breast pump system based on the output of the pattern recognition algorithm.

Most users will typically follow particular patterns when moving around. For example, when the user is about to run, they will typically have a motion pattern beforehand which can indicate that the user will begin running. As such, the measured motion [over time] can be input into a pattern recognition algorithm which can predict a future motion of the user based on the pattern(s) in the measured motion. This prediction can be used to adapt the breast pump system pre-emptively.

The controller may be further configured to transmit feedback to the user (e.g. via a display, a speaker system, a mobile phone etc.) indicative of the motion data of the user. For example, the controller may be further configured to transmit the feedback to the user when the motion data exceeds one or more thresholds (i.e. the change in motion and/or tilt is too high).

The system may further comprise an accelerometer, wherein the motion data comprises accelerometer sensor data from the accelerometer representative of the acceleration of the user.

The system may further comprise a gyroscope, wherein the motion data comprises gyroscope sensor data from the gyroscope representative of the orientation and/or angular acceleration of the user.

The gyroscopic data may comprise one or more of rotation data, position data relative to gravity and orientation relative to gravity.

The controller may be further configured to receive data corresponding to an amount of milk pumped by the breast pump system and adapt the one or more parameters based on the amount of milk pumped.

The more milk which has been pumped, the heavier the breast pump may be. As such, it may be beneficial to also adapt the parameters based on the amount of milk which has been pumped. The amount of milk which has been pumped could be obtained from volume and/or weight sensors in the breast pump. The amount of milk could also be estimated from the amount of time which the breast pump has been pumping, data from the pressure cycle and/or data from the pump motor.

The breast pump of the breast pump system may comprise an adaptable suction surface and the controller may be further configured to adapt the suction surface of the breast pump based on the motion data.

Increasing the pressure surface can provide a better hold for the breast pump, thereby decreasing the likelihood that the breast pump will disconnect or move from the user or leak.

The pressure surface may be the surface of the breast pump on which the pressure changes directly interact with the user. In other words, the pressure difference caused by the breast pump is acting on the pressure surface to generate the suction force.

The controller maybe adapted to increase the pressure surface in response to the motion data indicating an increase in motion of the user.

The suction force is proportional to both the absolute pressure difference and the suction surface. Thus, increasing the suction surface can also increase the suction force.

The system may further comprise the breast pump.

The system may further comprise a sensor system for obtaining the motion data. The sensor system may comprise an accelerometer and/or a gyroscope.

The invention also provides a method for controlling a suction force of a breast pump exerted on a user during operation, the method comprising:

   receiving and analyzing motion data of the user wearing the breast pump, the motion data being indicative of a motion intensity and/or a tilt of the user; and
   adapt one or more parameters of the breast pump during operation to adapt the suction force if the motion data indicates a change in motion intensity and/or tilt of the user.

The invention also provides a computer program product comprising computer program code which, when executed on a computing device having a processing system, cause the processing system to perform all of the steps of the afore-mentioned method.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a known breast pump system, comprising an expression unit and a drive arrangement for controlling the expression function of the breast pump;
Figure 2 shows accelerometer sensor data with motion intensity thresholds;
Figure 3 shows accelerometer sensor data of a user walking;
Figure 4 shows the root mean square (AcT) of the accelerometer sensor data;
Figure 5 shows the standard deviation of the AcT;
Figure 6 shows gyroscope sensor data for a user hanging up laundry;
Figure 7 shows the root mean square (GyT) of the gyroscope sensor data;
Figure 8 shows the standard deviation of GyT;
Figure 9 shows a conventional pressure profile for a breast pump;
Figure 10 shows a pressure profile with a baseline pressure differential;
Figure 11 shows a pressure profile with a shortened lower pressure differential time; and
Figure 12 shows a classification of acceleration intensity and the corresponding changes in the pressure profile.

DETAILED DESCRIPTION OF THE EMBODIMENTS

The invention will be described with reference to the Figures.
It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for controlling a holding force of a breast pump system exerted on a user during operation. A controller is used to receive and analyze motion data coming from sensors measuring the motion intensity and/or tilt of the user when wearing the breast pump system. If the motion data indicates a change in the motion intensity and/or the tilt of the user, one or more parameters of the breast pump system are adapted, during operation, to adapt the holding force exerted on the user.

For the sake of brevity, the following examples will be given with respect to a suction force exerted on a user by a breast pump. However, it will be appreciated that other holding forces exerted by other components of the breast pump system (e.g. a clamping-like force by the bra) could also be adapted as described below.

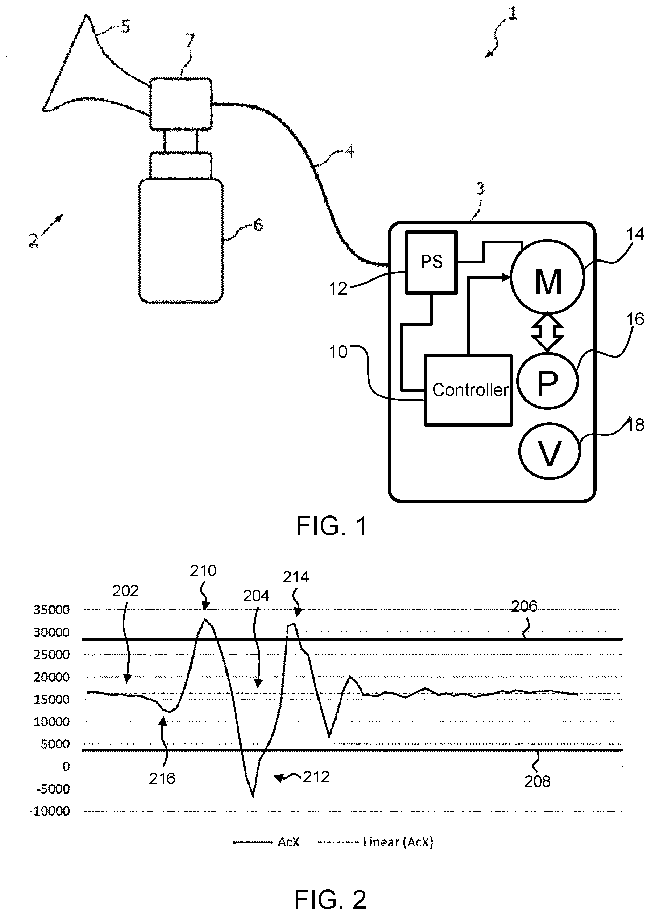

Figure 1 shows a known breast pump system 1, comprising an expression unit 2 and a drive arrangement for controlling the expression function of the breast pump.

In the example shown, the drive arrangement comprises an electric pump arrangement 3 which is connected via a tube 4 to the expression unit 2. The pump arrangement may include various components such as a pump impeller and the pump motor, so may be considered to be a general operating unit. Alternatively, or additionally, the pump arrangement may comprise a membrane pump, a displacement pump and/or a piston pump.

The expression unit 2 is formed with a main body 7, a funnel 5 (known as a breast shield) for receiving a breast of a user and a milk collection container 6 for collecting the expressed milk. The funnel 5 and the container 6 are connected to the main body 7. The main body 7 comprises a vacuum chamber. A flexible membrane known as the diaphragm is located in the vacuum chamber. The diaphragm prevents expressed milk from flowing into the tube 4 leading to the pump arrangement unit 3.

The operating unit, including the pump arrangement 3, may instead be directly mounted and connected to the main body 7. In this case, the diaphragm prevents expressed milk from flowing directly into the pump arrangement 3.

The pump arrangement 3 comprises a controller 10, a power source 12, a motor 14 and a vacuum pump 16. A membrane pump (displacement pump) may instead be used. The controller controls 10 the operation of the power source 12, motor 14 and vacuum pump 16. The pump arrangement 3 further comprises a solenoid valve 18.

In use, the vacuum pump 16 applies a vacuum to the diaphragm located in the main body 7 so that it deforms. The diaphragm deforms to create a vacuum in the funnel 5, when it is sealed to the breast, which in turn applies a vacuum (i.e. a negative pressure relative to atmospheric pressure) to the breast which enables milk to be expressed.

The vacuum is applied to the breast at intervals. That is, a pressure differential is applied on a cyclic basis. The particular pressure differences applied over time may be referred to as a pressure profile of the breast pump. After a vacuum has been established, the pressure from the vacuum is released by the use of the solenoid valve which is temporarily opened. The solenoid valve is an electromechanically operated valve configured to open and close an air passage that connects the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection container. This enables breast pumping to be performed more discretely and leaves the hands free to do other things.

There are also other types of wearable breast pump whereby the expression unit is fixed to a breast and the pump unit and/or milk collection container are situated elsewhere on the body of a user.

The invention provides a wearable breast pump that adjusts the pressure profile of the breast pump based on the movements and rotations of the user such that the breast pump stays in place even when sudden movements or rotations (i.e., high accelerations) occur by the user. This prevents the pump from moving and/or leaking.

For example, a system is provided comprising a wearable breast pump, including a vacuum pump, a sensor unit (e.g., including an accelerometer or gyroscope), which detects movements and/or rotations by the user and a controller which adjusts the pressure profile of the pump based on the measured movements and/or orientation from the sensor unit.

The controller may be the controller 10 provided in the known breast pump system 1 or may be separate to the breast pump system 1. For example, the breast pump system 1 may be connected to a mobile phone such that the mobile phone controls the pressure profile of the breast pump system 1.

In some cases, the sensor unit could be a sensor unit provided on a smart phone. Current smart phones tend to come with sensors such as accelerometers and gyroscopes. These could be used as the afore-mentioned sensor unit. For example, when the user is holding the smart phone or has the smart phone in their pocket/bag etc., the sensor data from the sensors in the smart phone correspond to the movement of the user.

Figure 2 shows accelerometer sensor data with motion intensity thresholds. In this example, a sensor unit detects the movements of the user with an accelerometer. The solid line 202 shows the output of the accelerometer over time. The x-axis represents time (s) and the y-axis represents the intensity of acceleration. In this example (and all graphs shown below) the numerical values of the acceleration intensity are shown merely for context.

Based on the signal 202 (AcX), a linear trend line 204 (Linear(AcX)) can be calculated, which makes it possible to determine an upper threshold (206) and a lower threshold (208). The controller can thus adjust the pressure profile of the pump when a user's acceleration exceeds the predefined thresholds. Alternatively, a relationship between acceleration intensity and the pressure profile could be used to apply different pressure profiles for different acceleration levels.

It is noted that the pressure profile is defined by one or more parameters of the breast pump. These will be further discussed below.

Three acceleration peaks 210, 212 and 214 can be seen in signal 202. These peaks exceed the thresholds 206 and 208 and thus indicate a sudden movement. As such, the pressure profile can be adapted when these peaks occur.

The sensor unit can send the accelerometer sensor data to the controller. Once the controller receives the accelerometer sensor data, it can analyze the data (e.g., compare it to the thresholds or to pre-defined relationship) and adapt the pressure profile accordingly.

In another example, the controller may be configured to predict the subsequent acceleration based on a deviation from the linear trend line 204 and adjust the pressure profile accordingly. For example, the subtle motion shown at point 216 in Figure 2 could indicate a sudden movement will occur in the future. This allows for the prediction of sudden high accelerations and the pre-emptive adaptation of the pressure profile.

Models for recognition and classification of patterns in the motion performed by the user could also be used to enable the pre-emptive adjustment of the pressure profile in such a way that the highest suction force is reached at the highest acceleration of the pump.

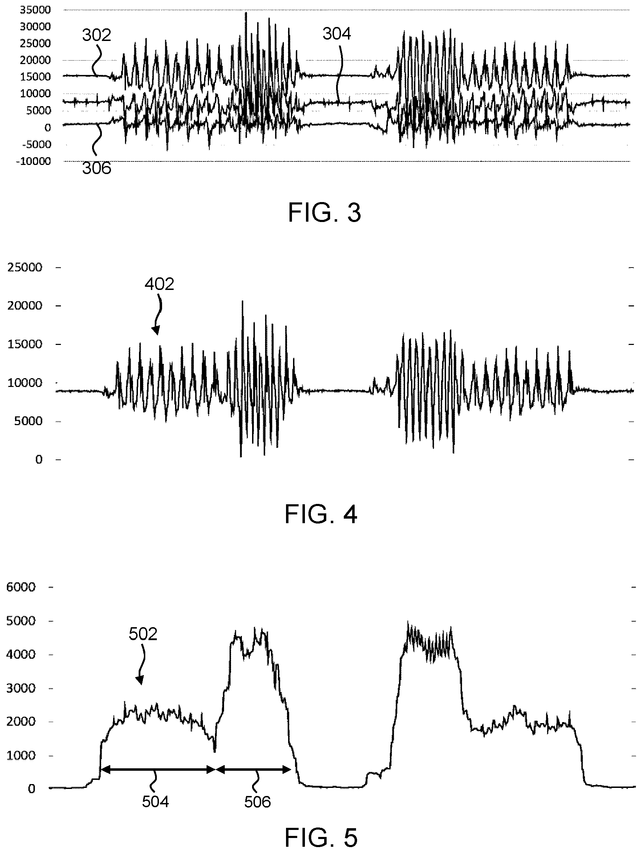

Figure 3 shows accelerometer sensor data of a user walking. In particular, Figure 3 shows accelerometer sensor data of a user first walking straight, walking down the stairs, turning around and then walking up the same stairs again. The accelerometer sensor was placed on the left breast of the user when obtaining the data of Figure 3.

The x-axis represents time (s) and the y-axis represents an acceleration intensity. Line 302 shows the acceleration intensity over time in the X direction (AcX), line 304 shows the acceleration intensity over time in the Y direction (AcY) and line 306 shows the acceleration intensity over time in the Z direction (AcZ).

In some cases (e.g. walking, walking up stairs etc.), the raw acceleration values may not provide enough context to enable the pressure profile to adapt appropriately. For example, the cyclic nature of the acceleration intensity when walking may mean that the pressure profile is cyclically adapted, which may not be comfortable for the user.

Figure 4 shows the root mean square of the accelerometer sensor data. The root mean square can be calculated as such:

$$AcT = \sqrt{\frac{(AcX^2 + AcY^2 + AcZ^2)}{3}}$$

Where AcT is the root mean square of the accelerometer sensor. This provides a combination of the magnitudes of all three accelerometer sensor signals data (AcX, AcY and AcZ.

AcT is plotted as line 402. This provides further context to the motion of the user compared to the original accelerometer sensor data. However, the AcT is still cyclic and thus may not be the best option to interpret the motion of the user. For even further context, the standard deviation of AcT can be calculated for a defined period of time.

Figure 5 shows the standard deviation of the AcT. The standard deviation (with time period of less than one second) is shown in line 502, where the x-axis represents time (s) and y-axis represents the acceleration intensity. As shown in Figure 5, the standard deviation enables an improved distinction between high intensity pattern movements such as walking and walking up and down stairs. For example, it now becomes clearer that the user was walking during time period 504 and the user was walking down the stairs during time period 506. Additionally, the use of the standard deviation makes it possible to set a single threshold for movement patterns that cause relatively high accelerations (as there are no negative peaks).

It is noted that the time period of below one second was used for the standard deviation as this provides an adequate time to detect sudden accelerations in time. Of course, other time periods could be used depending on the particular actions expected from the user.

In addition to, or as an alternative to, the accelerometer, the sensor unit could comprise a gyroscope to detect the angular acceleration and/or orientation of the user and adjust the pressure profile when the user is suddenly rotating left/right or tilting forward (e.g., when the user bends forward). In addition, the controller could indicate that the user is tilting too much forward by providing feedback to the user (e.g. haptic or sound feedback).

The accelerometer and/or the gyroscope may be placed on, or near, the breast pump to provide information on the acceleration/rotation of the breast pump induced by the motion of the user.

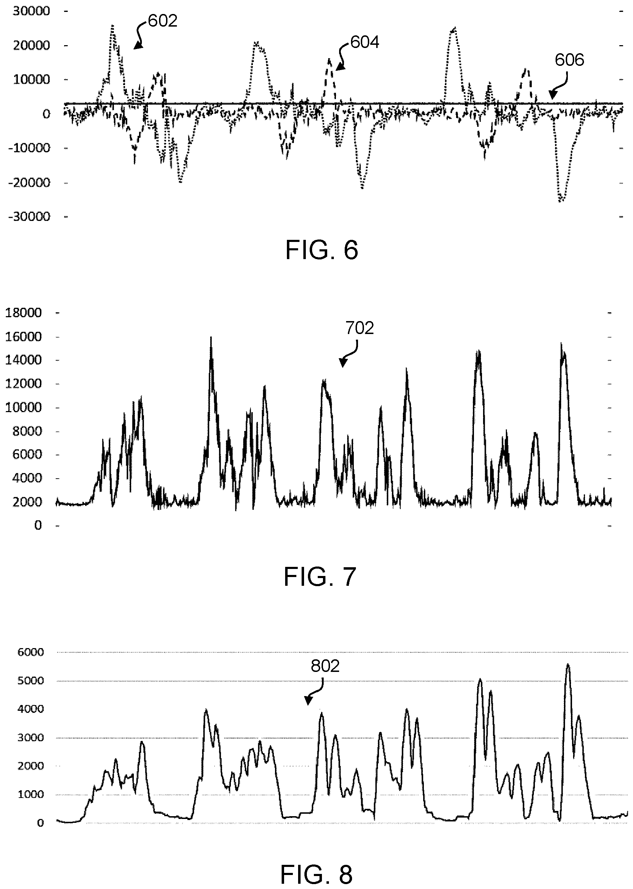

Figure 6 shows gyroscope sensor data for a user hanging up laundry. The x-axis represents time (s) and the y-axis represents the angular acceleration of the user with respect to one of three gyroscopic axes. Dotted line 602 shows the angular acceleration of the user with respect to the Y axis of the gyroscope (GyY), dashed line 604 shows the angular acceleration of the user with respect to the Z axis of the gyroscope (GyZ) and the solid line 606 shows the angular acceleration of the user with respect to the X axis of the gyroscope (GyX).

As will be appreciated, a non-zero angular acceleration results in a change in orientation of the user. A typical action which results in inadequate suction forces occurs when the user is tilting (e.g. forwards). This, in essence, is a change in orientation which can be measured from the gyroscope sensor data.

The gyroscope sensor data is representative of the user turning to the left, bending down, coming back up, turning to the right and then hanging the laundry on the drying rack. This action is repeated three times by the user. The gyroscope sensor was placed on the left breast of the user.

Similarly to the accelerometer sensor data, by calculating the root mean square of the gyroscope sensor data, it is more convenient to associate meaningful context information to the data. As before, the root mean square is determined as such:

$$GyT = \sqrt{\frac{(GyX^2 + GyY^2 + GyZ^2)}{3}}$$

Where GyT is the root mean square of the gyroscope sensor data. This provides a combination of the magnitudes of all three gyroscope sensor signals.

Figure 7 shows the root mean square of the gyroscope sensor data. The x-axis represents time (s) and the y-axis represents an angular acceleration. The line 702 shows the root mean square of the gyroscope sensor data (i.e., GyT). As before, the standard deviation of GyT can also be determined for even further context.

Figure 8 shows the standard deviation of GyT. The x-axis represents time (s) and the y-axis represents an angular acceleration. The standard deviation was calculated with a time period of less than one second in order to distinguish high intensity pattern movements, such as rotating and tilting. As before, the determination of the standard deviation makes it possible to set a single threshold for rotation patterns that cause high angular accelerations.

As previously mentioned, the accelerometer sensor data and/or the gyroscope sensor data can be used to adapt the pressure profile. Of course, other types of sensors which can also measure motion intensity and/or tilt, or even detect sudden changes in the motion intensity or tilt can be used. For example, a sensor which provides the orientation of the user (e.g. relative to gravity) can be used such that, when the orientation of the user exceeds a tilting threshold, the pressure profile of the breast pump is adapted to increase the suction force.

Other sensor could also be used to indicate of a motion intensity and/or a tilt of the user. Microphones could be used to hear and detect when there is activity (i.e. motion) from the user. Location sensors could also be used to predict activity from the user. Of course, it will be appreciated that many different types of sensors (and combinations thereof) could be used to indicate the motion intensity and/or tilt of the user.

The pressure profile is, in essence, comprised of the parameters which define how the pump of the breast pump system operates. Breast pumps make use of an alternating negative pressure which is applied to the part of the breast and nipple within one, or two, breast shields to draw the milk from the nipple into a collection vessel. Typical pressure profiles oscillate between a minimum absolute pressure and then return to atmospheric pressure (or near atmospheric pressure) at the end of each cycle.

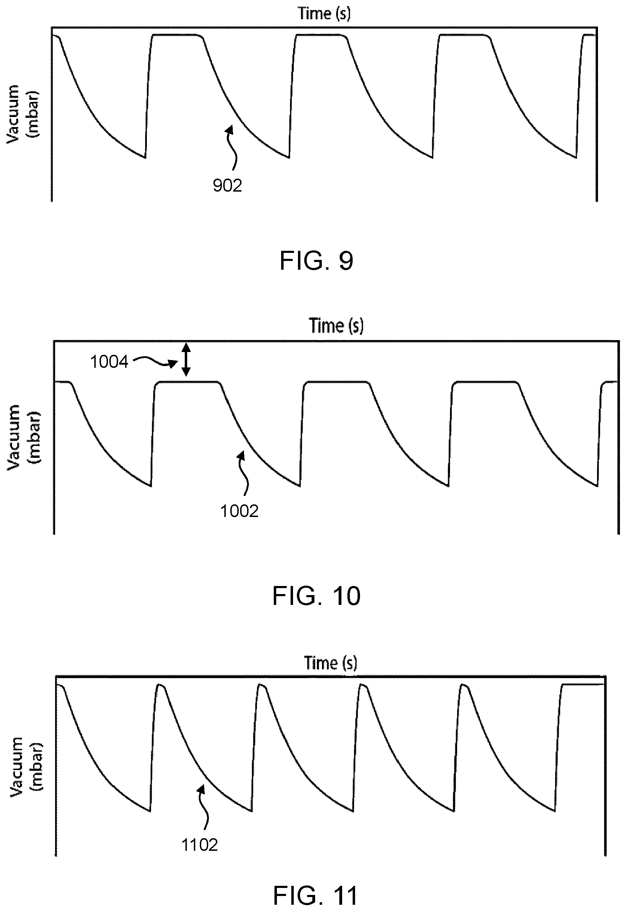

Figure 9 shows a conventional pressure profile for a breast pump. The x-axis represents time (s) and the y-axis represents the pressure in the breast shield. The top of the y-axis represents atmospheric pressure. At the start of a pumping session, the pressure in the interface is equal or close to the atmospheric pressure (e.g. around 1000 mBar absolute pressure). In most breast pumps, the pressure then goes down to about 300 to 350 mBar below atmospheric pressure in the first part of the pressure cycle. In the second part of the cycle, a pressure release valve is opened to let in air and the pressure raises again to a pressure that is close to atmosphere. This typical cyclic pressure profile is shown in line 902.

However, a return to ambient/atmospheric pressure within the breast shield chamber may not be required as it has been found, from research, that babies maintain a baseline vacuum during feeding. The function of the baseline vacuum in a baby's mouth is to keep a good latch on the mother (i.e., to keep the breast/nipple at a stable position). These benefits, as well as the benefit of keeping the breast pump in position, can also be achieved by maintaining a minimum level of differential pressure on the breast throughout at least a portion of the pumping session when using a breast pump.

Figure 10 shows a pressure profile with a baseline pressure difference. The x-axis represents time (s) and the y-axis represents the pressure in the breast shield. The top of the y-axis represents atmospheric pressure. A pressure difference is a difference between atmospheric pressure and the (negative) pressure induced by the breast pump. The cyclic pressure, with a baseline pressure difference 1004 (relative to atmospheric pressure), is shown in line 1002. This creates a sort of vacuum in the breast shield, holding the breast pump against the user.

The pressure profile shown in Figure 10 is an exemplary pressure profile to which the breast pump could be adapted when the user accelerates above a motion threshold or when the orientation of the user (relative to gravity) exceeds a tilting threshold.

When the user is moving around, thereby inducing higher acceleration forces, the baseline pressure difference will help (as it does with babies) to keep the breast pump/breast/nipple positioned and avoid leaks.

This is because the use of a baseline pressure difference ensures that there is always a minimum suction force on the baby pump towards the breast of the user at all times during the pressure cycle which can overcome the acceleration forces induced the motion of the user.

For example, the baseline pressure may be between 10 mBar and 150 mBar below atmospheric pressure. For example, the baseline pressure may be 10, 20, 30, 40, 50, 70, 100, 150 mBar below atmospheric pressure. Other values could also be used.

As an alternative to, or in addition to, providing a baseline pressure difference, the controller could shorten the time that the pressure, during the pressure cycle, is at the lower pressure difference (i.e., closer to atmospheric).

Figure 11 shows a pressure profile with a shortened lower pressure difference time. The x-axis represents time (s) and the y-axis represents the pressure in the breast shield. The top of the y-axis represents atmospheric pressure. The cyclic pressure profile with a shorter time at the lower pressure difference is shown in line 1102.

The pressure profile shown in Figure 11 is an exemplary pressure profile to which the breast pump could be adapted when the user accelerates above a motion threshold or when the orientation of the user (relative to gravity) exceeds a tilting threshold.

The shorter time spent at the lower pressure difference means that there is less time for the breast pump to de-attach or slip from the user. This is because, at lower pressure differentials, the breast pump has a lower suction force acting on it and thus is more susceptible to de-attaching from the user when the user suddenly accelerates.

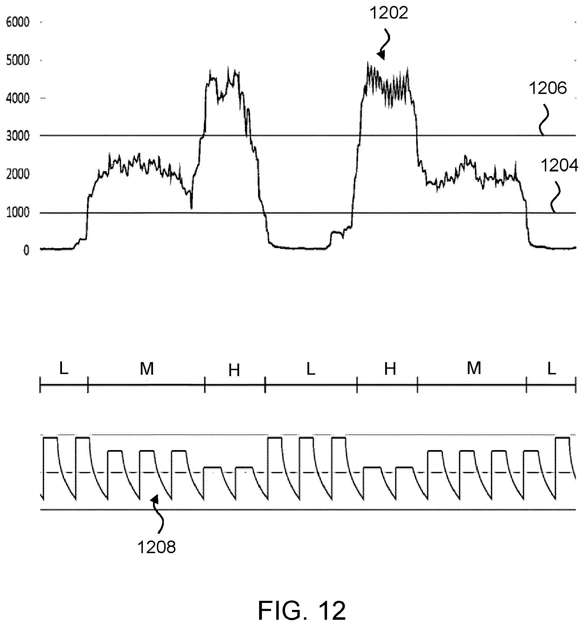

Figure 12 shows a classification of acceleration intensity and the corresponding changes in the pressure profile. The top graph shows the acceleration intensity (y-axis) versus time (x-axis). Line 1202 shows the standard deviation of accelerometer sensor data which was previously shown in Figure 5. Additionally, two thresholds are shown in the graph. The first threshold 1204 indicates a "medium" level of activity (M) and the second threshold 1206 indicates a "high" level of activity (H) from the user. Similarly, any acceleration values below the first threshold 1204 indicate a "low" level of activity (L), or, being at rest.

Each classification relates to a different pressure profile as shown in the bottom graph. The bottom graph shows an adaptive pressure profile where the x-axis represents time (s) and the y-axis represents the absolute pressure (with atmospheric pressure being at the top of the y-axis). In this case, the medium (M) and high (H) classification cause the baseline pressure to decrease, thereby increasing the pressure differential relative to atmospheric pressure, as can be seen by the changes in line 1208 which correspond to line 1202 crossing the thresholds.

It is noted that the term "suction force" is used broadly throughout the specification. Of course, in practice, the suction force depends on the particular pressure difference. However, for the sake of this specification, the suction force can be treated as an "overall" suction force (e.g. mean/average suction force over time, total suction force during a complete cycle of the pressure profile etc.). This is because reducing the time which the breast pump spends at the lower pressure differential does not necessarily change the specific suction force during the lower pressure differences, but it does change the overall suction force over time (e.g. during a full cycle).

Thus, the advantages of the examples provided herein can be achieved by increasing the minimum suction force (in the pressure cycle) and/or reducing the duration of time spent at the minimum suction force.

The adjustments in the pressure profile (e.g. pressure differential needed for a sudden high acceleration of the user) can also be adjusted based on the volume of milk which has been pumped. This is because the breast pump may get heavier when more milk is stored in it and thus a larger force may be needed to prevent high accelerations induced by the user causing the breast pump to detach from the user.

The suction force is proportional to the pressure difference and the area on which the pressure difference is acting. Thus, another option to increase the suction force could be to increase the pressure surface around the areola region on which the pressure difference is acting. This could enable the breast pump to stay attached to the breast without having to increase the pressure difference to uncomfortable values for the user.

In another embodiment, the wearable breast pump is placed in a smart bra with self adjusting straps which adapt based on the motion intensity and/or tilt of the user. Thus, a holding force which holds the breast pump against the user can be increased by adapting the straps of the bra.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A system for controlling a holding force of a breast pump system exerted on a user during operation, the system comprising a controller configured to:

   receive and analyze motion data (202, 302, 304, 306, 602, 604, 606) of the user wearing the breast pump system, the motion data being indicative of a motion intensity and/or a tilt of the user; and
   adapt one or more parameters of the breast pump system during operation to adapt the holding force if the motion data indicates a change in motion intensity and/or tilt of the user.

2. The system of claim 1, wherein the controller is configured to adapt one or more parameters of the breast pump system during operation to increase the holding force if the motion data indicates an increase in motion intensity of the user and/or an increase in the tilt of the user relative to gravity.

3. The system of claims 1 or 2, wherein the controller is configured to adapt one or more parameters of the breast pump system during operation to decrease the holding force if the motion data indicates a decrease in motion intensity of the user and/or a decrease in the tilt of the user relative to gravity.

4. The system of any one of claims 1 to 3, wherein the breast pump system comprises a breast pump and wherein the holding force is comprised of at least a suction force exerted on the user.

5. The system of claim 4, wherein the breast pump operates by cycling between a minimum absolute pressure and a baseline pressure and wherein, in adapting one or more of the parameters of the breast pump, the controller is configured to adapt the baseline pressure.

6. The system of claim 5, wherein, in adapting the baseline pressure, the controller is configured to adapt a magnitude of the baseline pressure to increase an absolute pressure difference between ambient pressure and the baseline pressure.

7. The system of claims 5 or 6, wherein, in adapting the baseline pressure, the controller is configured to decrease a time spent at the baseline pressure during a cycle.

8. The system of any one of claims 5 to 7, wherein the breast pump comprises an adaptable suction surface and the controller is further configured to adapt the suction surface of the breast pump based on the motion data.

9. The system of any one of claims 1 to 8, wherein the controller is configured to adapt one or more of the parameters of the breast pump system when the motion data indicates that the change in motion intensity of the user exceeds a motion intensity threshold and/or that the tilt of the user relative to gravity exceeds a tilt threshold.

10. The system of claim 9, wherein the controller is configured to provide feedback to the user when the motion data indicates that the change in motion intensity of the user exceeds a motion intensity threshold and/or that the tilt of the user relative to gravity exceeds a tilt threshold.

11. The system of any one of claims 1 to 10, wherein the controller is further configured to predict a change in motion intensity and/or a change in tilt based on the motion data and adapt one or more of the parameters of the breast pump system pre-emptively based on the predicted change in motion intensity and/or the predicted change in tilt.

12. The system of any one of claims 1 to 11, further comprising:

    an accelerometer, wherein the motion data comprises accelerometer sensor data from the accelerometer representative of the acceleration of the user; and/or
    a gyroscope, wherein the motion data comprises gyroscope sensor data from the gyroscope representative of the orientation and/or angular acceleration of the user.

13. The system of any one of claims 1 to 12, wherein the controller is further configured to receive data corresponding to an amount of milk pumped by the breast pump system and adapt the one or more parameters based on the amount of milk pumped.

14. A method for controlling a holding force of a breast pump system exerted on a user during operation, the method comprising:

    receiving and analyzing motion data (202, 302, 304, 306, 602, 604, 606) of the user wearing the breast pump system, the motion data being indicative of a motion intensity and/or a tilt of the user; and
    adapt one or more parameters of the breast pump system during operation to adapt the holding force if the motion data indicates a change in motion intensity and/or tilt of the user.

15. A computer program product comprising computer program code which, when executed on a computing device having a processing system, cause the processing system to perform all of the steps of the method according to claim 14.

FIG. 1

FIG. 2

FIG. 3

FIG. 4

FIG. 5

FIG. 6

FIG. 7

FIG. 8

Time (s)

Vacuum (mbar)

902

FIG. 9

Time (s)

Vacuum (mbar)

1004

1002

FIG. 10

Time (s)

Vacuum (mbar)

1102

FIG. 11

FIG. 12

Europäisches
Patentamt

European
Patent Office

Office européen
des brevets

**EUROPEAN SEARCH REPORT**

Application Number

EP 22 18 3025

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/125569 A1 (WILLOW INNOVATIONS INC [US]) 16 June 2022 (2022-06-16) | 1,4-7,9, 10,12-15 | INV. A61M1/06 |
| Y | * paragraphs [0047] - [0084]; figures 1, 2 | 8 | |
| A | * | 2,3,11 | |
| | ----- | | |
| A | US 2021/205513 A1 (O'TOOLE JONATHAN [GB] ET AL) 8 July 2021 (2021-07-08) * paragraphs [0209], [0242], [0669]; figure 1 * | 1,14,15 | |
| | ----- | | |
| Y | EP 4 000 661 A1 (KONINKLIJKE PHILIPS NV [NL]) 25 May 2022 (2022-05-25) | 8 | |
| A | * paragraphs [0042] - [0063]; figures 1-6 * | 1,14,15 | |
| | ----- | | |

TECHNICAL FIELDS SEARCHED (IPC)

A61M

The present search report has been drawn up for all claims

| Place of search | Date of completion of the search | Examiner |
|---|---|---|
| The Hague | 4 November 2022 | Schlaug, Martin |

## ANNEX TO THE EUROPEAN SEARCH REPORT
## ON EUROPEAN PATENT APPLICATION NO.

EP 22 18 3025

This annex lists the patent family members relating to the patent documents cited in the above-mentioned European search report.
The members are as contained in the European Patent Office EDP file on
The European Patent Office is in no way liable for these particulars which are merely given for the purpose of information.

04-11-2022

| Patent document cited in search report | Publication date | Patent family member(s) | | Publication date |
|---|---|---|---|---|
| WO 2022125569 A1 | 16-06-2022 | NONE | | |
| US 2021205513 A1 | 08-07-2021 | AU | 2018283715 A1 | 16-01-2020 |
| | | CA | 3066971 A1 | 20-12-2018 |
| | | CN | 213964594 U | 17-08-2021 |
| | | CN | 216798371 U | 24-06-2022 |
| | | EP | 3638334 A1 | 22-04-2020 |
| | | EP | 4066870 A2 | 05-10-2022 |
| | | JP | 2020523179 A | 06-08-2020 |
| | | SG | 11201912135S A | 30-01-2020 |
| | | US | 2018361040 A1 | 20-12-2018 |
| | | US | 2020139026 A1 | 07-05-2020 |
| | | US | 2021170080 A1 | 10-06-2021 |
| | | US | 2021196873 A1 | 01-07-2021 |
| | | US | 2021196874 A1 | 01-07-2021 |
| | | US | 2021196875 A1 | 01-07-2021 |
| | | US | 2021196876 A1 | 01-07-2021 |
| | | US | 2021205511 A1 | 08-07-2021 |
| | | US | 2021205512 A1 | 08-07-2021 |
| | | US | 2021205513 A1 | 08-07-2021 |
| | | US | 2021205514 A1 | 08-07-2021 |
| | | US | 2021205515 A1 | 08-07-2021 |
| | | US | 2021205516 A1 | 08-07-2021 |
| | | US | 2021205517 A1 | 08-07-2021 |
| | | US | 2021205518 A1 | 08-07-2021 |
| | | US | 2021228789 A1 | 29-07-2021 |
| | | US | 2021268158 A1 | 02-09-2021 |
| | | WO | 2018229504 A1 | 20-12-2018 |
| EP 4000661 A1 | 25-05-2022 | EP | 4000661 A1 | 25-05-2022 |
| | | WO | 2022106252 A1 | 27-05-2022 |

EPO FORM P0459

For more details about this annex : see Official Journal of the European Patent Office, No. 12/82